# EUROPÄISCHE PATENTANMELDUNG

(11) **EP 2 078 518 A2**
(43) Veröffentlichungstag der Anmeldung: **15.07.2009**
(21) Anmeldenummer: 08020363.1
(22) Anmeldetag: 22.11.2008
(51) Int. Cl.: A61F 13/08

(54) **Kompressions- oder Stützstrumpf**

(30) Priorität: 28.12.2007 DE 102007063568
(71) Anmelder: Paul Hartmann Aktiengesellschaft, 89522 Heidenheim (DE)
(72) Erfinder: Virkus, Antje, 67000 Strasbourg (FR); Berndt, Erik, 89542 Herbrechtingen (DE)
(74) Vertreter: Langöhrig, Angelika Beate

(57) **Zusammenfassung**

Die Erfindung betrifft einen Kompressions- oder Stützstrumpf (10) zum Anlegen an ein menschliches Bein umfassend einen röhrenförmigen komprimierenden Beinabschnitt (12) aus einem elastischen Material, der das Bein eines Trägers umschließt sowie einen Fersenabschnitt (13), der sich mittelbar oder unmittelbar an den komprimierenden Beinabschnitt (12) distal anschließt, wobei ein mittelbar oder unmittelbar an den Fersenabschnitt (17) distal anschließender röhrenförmiger erster Bundabschnitt (18) zur Fixierung der Lage des Kompressions- oder Stützstrumpfes (10) im Bereich eines Fußes des Trägers vorgesehen ist, wobei der erste Bundabschnitt (18) im Bereich der Längsgewölbe (24) oder proximal der Längsgewölbe (24) eines Fußes des Trägers zu liegen kommt.

## Beschreibung

Die Erfindung betrifft einen Kompressions- oder Stützstrumpf zum Anlegen an ein menschliches Bein umfassend einen röhrenförmigen komprimierenden Beinabschnitt aus einem elastischen Material, der das Bein eines Trägers umschließt, sowie einen Fersenabschnitt, der sich unmittelbar oder mittelbar an den komprimierenden Beinabschnitt distal anschließt.

Kompressions- und Stützstrümpfe werden zum einen in der medizinischen Kompressionstherapie und zum anderen im nicht therapeutischen, sondern kosmetischen Bereich als Stützstrümpfe verwendet, um einen Ruhe- sowie Arbeitsdruck auf ein menschliches Bein aufzubringen. Unter dem Ruhedruck wird dabei der Druck verstanden, den der Strumpf als solcher auf das ruhende Bein aufbringt, wobei der Arbeitsdruck der Druck ist, der beim Bewegen des Beins durch die Kontraktion des Muskels und den Strumpf auf das Bein ausgeübt wird.

Dabei dienen derartige Kompressions- und Stützstrümpfe zum einen zur Behandlung einer Vielzahl venöser Leiden und werden auch nach beispielsweise Veneneingriffen, wie Stripping, Schaumsklerosierung oder auch Laserinterventionen, eingesetzt.

Dabei sind medizinische Kompressionsstrümpfe zur Gütesicherung in der Norm RAL-GZ 387 vom September 2000 des Deutschen Instituts für Gütesicherung und Kennzeichnung e.V. beschrieben.

Ein gattungsgemäßer Kompressions- oder Stützstrumpf ist dabei beispielsweise aus der EP 0 927 014 B1 bekannt, die eine Kompressionsorthese vom Typ eines Stiefels für das Fixieren des Beins nach einem venösen Geschwür beschreibt, wobei an den Beinabschnitt ein röhrenförmiger nicht komprimierender Fußabschnitt anschließt, der den Fuß bis zur Wurzel der Zehen überdeckt, ohne ihn zu komprimieren und ohne die Bildung von Falten.

Daneben beschreibt die GB-PS 449 659 einen gamaschenartigen Stützstrumpf, ebenso wie die WO 02/064073 A1, die eine Stützstrumpfhose insbesondere mit einem den Fuß umgreifenden Steg beschreibt, an dem wahlweise jedoch auch ein Fußteil bis zu den Zehen angebracht sein kann.

Daneben existieren auch Stütz- oder Kompressionsstrümpfe, die lediglich gamaschenartig den Unterschenkel und/oder den Oberschenkel umschließen, wobei derartige Kompressionsstrümpfe in der EP 0 904 040 B1 sowie US-PS 4,513,740 beschrieben sind.

Ausgehend von diesem Stand der Technik soll es nun Aufgabe der Erfindung sein, einen Kompressionsstrumpf bereitzustellen, bei dem ein Abschnüren der Zehe, wie es insbesondere bei Strümpfen mit offenem Fußteil passieren kann, verhindert wird, wobei der Strumpf gleichzeitig einen hohen Tragekomfort aufweist.

Die Erfindung löst diese Aufgabe durch einen Kompressions- oder Stützstrumpf der gattungsgemäßen Art, bei dem ein mittelbar oder unmittelbar an den Fersenabschnitt distal anschließender röhrenförmiger erster Bundabschnitt zur Fixierung der Lage des Kompressions- oder Stützstrumpfes im Bereich des Fußes eines Trägers vorgesehen ist, wobei der Bundabschnitt im Bereich der Längsgewölbe oder proximal der Längsgewölbe eines Fußes des Trägers zu liegen kommt. Die Lage bezieht sich dabei insbesondere auf ein inneres Längsgewölbe des Fußes.

Gemäß der vorliegenden Erfindung soll dabei unter einem Bundabschnitt ein Abschluss eines Strumpfes verstanden sein. Dieser Abschluss kann durch jede bekannte Technik, wie beispielsweise Doppeltlegen des Strumpfmaterials oder Ansticken oder Annähen eines weiteren Materials gebildet werden.

Eine derartige Gestaltung besitzt den Vorteil eines Strumpfes, der bei gleichem Kompressionsgrad wie herkömmliche Strümpfe mit einem Fußteil, an den sich dann der Bundabschnitt im Bereich des Ballens oder der Zehen anschließt, einen höheren Tragekomfort besitzt. Im angelegten Zustand kann so ein Abschnüren der Zehen weitgehend unterbunden werden. Darüber hinaus besitzt ein derartiger Kompressionsstrumpf den Vorteil, auch mit im Fußbereich offenen Schuhen getragen werden zu können, wie sie im Sommer vielfach verwendet werden. Aus diesem Grund tragen viele betroffene Personen Kompressionsstrümpfe im Sommer sehr ungern oder gar nicht, da sie einerseits glauben, dass die Strümpfe zu dick seien und dass man deswegen anfange zu schwitzen, und andererseits, dass sie gerade mit modischem Schuhwerk mit offener Schuhspitze wie beispielsweise Sandalen mit Kompressionsstrümpfen ein hässliches Erscheinungsbild ergäben. Dabei ist gerade im Sommer die Belastung auf die Venen sehr hoch, weshalb das Tragen von Kompressionsstrümpfen häufig gerade in diesen Monaten erforderlich wäre. Dieser Konflikt kann mit dem erfindungsgemäßen Strumpf gelöst werden.

Darüber hinaus besitzt ein derartiger Strumpf den Vorteil, dass durch den freien Fuß- und Zehenteil auch ältere Patienten, die oftmals Fuß- bzw. Zehenstellungsprobleme haben und daher keine Kompression im Fuß- oder Zehenteil vertragen, einen derartigen Kompressions- oder Stützstrumpf problemlos zu tragen vermögen. Das Gleiche gilt auch, sofern der Träger, insbesondere in Folge einer Diabetes unter Wunden im Zehenbereich leidet, so dass im Zehenbereich geschlossen Strümpfe nicht tragbar sind.

Auf der anderen Seite vermag ein erfindungsgemäßer Strumpf mit Ferse den Druck ausgehend vom Beinabschnitt besser zu verteilen, als ein gamaschenartiger Strumpf und ist auch angenehm zu tragen und leichter in der richtigen Position anzulegen. Durch den erfindungsgemäßen Strumpf wird letztendlich auch die Anatomie eines Fußes besser
berücksichtigt.

Dabei weist jeder Fuß zwei Längsgewölbe sowie ein Quergewölbe auf, wodurch das Körpergewicht hauptsächlich über die drei Punkte Ferse, Großzehengelenk (Großzehenballen) und Kleinzehengelenk (Kleinzehenballen) getragen wird. Die Fußgewölbe werden dabei durch Muskulatur verspannt und durch Bänder aufrechterhalten. Dabei spricht man insbesondere von einem mittleren oder inneren Längsgewölbe, das oft auch als Hauptgewölbe bezeichnet wird. Dieses Gewölbe verläuft entlang des Ristes und ist wichtig für die Erhaltung des Gleichgewichts, es gibt Schwung für die Vorwärtsbewegung beim Gehen und es fängt die Hauptlast der Stöße ab, denen der Fuß ausgesetzt ist. Dieses Längsgewölbe erstreckt sich im Wesentlichen zwischen dem Großzehnballen und der Ferse.

Darüber hinaus existiert ein seitliches oder äußeres Längsgewölbe, das manchmal auch als Außengewölbe bezeichnet wird. Es ist nur ein sehr schwaches Gewölbe, das als Stabilisator für das Gleichgewicht des Fußes fungiert und sich insbesondere zwischen der Ferse und dem Ballen im Bereich des kleinen Zehs erstreckt.

Das vordere Mittelfuß- oder Quergewölbe verläuft im rechten Winkel zu den beiden anderen Gewölben und gibt quer über den ganzen Fuß hinweg Unterstützung und Gleichgewicht. Es verläuft dabei proximal des Großzehn- und Kleinzehnballens.

Die Konstruktion der Gewölbe wird von den Formen und Gelenken der darin enthaltenen Knochen aufrechterhalten sowie durch ein Netzwerk aus Bändern, die die Knochen zusammenhalten. Darüber hinaus spielen auch die Muskeln und Sehnen eine wichtige Rolle bei der Unterstützung der Gewölbe. Insbesondere für die Aufrechterhaltung des Längsgewölbes sind die Fußsohlensehnenplatte und das lange Sohlenband wichtig. Die Fußgewölbe haben dabei keinen oder keinen wesentlichen Kontakt zum Boden. Dadurch, dass der erste Bundabschnitt im Bereich der Längsgewölbe des Fußes zu liegen kommt, besteht der Vorteil, dass nur ein geringer Kontakt des ersten Bundabschnitts mit dem Boden besteht, wodurch weitgehend verhindert werden kann, dass es durch den Bodenkontakt beim Laufen zu einem Verrutschen oder zu einem Umschlagen oder Aufrollen des Bundabschnitts kommt, der das Tragen unkomfortabel macht und einen sicheren Halt eines entsprechenden Kompressionsstrumpfes erschwert.

Kompressionsstrümpfe dienen dabei vielfach zur Therapie einer venösen Insuffizienz. Grundsätzlich kann die chronische venöse Insuffizienz nach Wiedmer in drei Stadien eingeteilt werden. Demnach wird die chronische venöse Insuffizienz unterschieden in:
- Grad 1 der chronischen venösen Insuffizienz ist demnach durch um die Knöchel und oberhalb des Fußgelenks angeordnete, besenreißerartige Venen (Corona phlebectatica) und Knöchelödeme gekennzeichnet.
- Grad 2 äußert sich durch die Hyperpigmentierung der Haut, Unterschenkelödeme und Dermatoliposklerose. Die Haut ist fest mit der Fascia curis verbacken, nicht in Falten abhebbar und zeigt vermehrten Glanz. Als Extremvariante der Dermatoliposklerose gilt die
   Atrophie blanche (auch als Capillaritis alba bezeichnet), die fast ausschließlich als Folge einer chronischen venösen Insuffizienz vorkommt und sich durch Depigmentierung im distalen Unterschenkel infolge Vasculitis kleiner Hautgefäße zeigt, die oft eine schmerzhafte Vorstufe des Ulcus cruris ist.
   Charakteristisch für diese Hautveränderung sind weiße, atrophische, münz- bis handtellergroße Herde. Sie sind bevorzugt in der Knöchelregion bzw. im Narbenbereich abgeheilter Ulcerationen lokalisiert.
- Grad 3 manifestiert sich als florides oder abgeheiltes Ulcus cruris venosum. Es hat als Prädilektionsstelle die perimalleoläre Region (Bisgaard'sche Kulisse), kann jedoch auch an anderen Stellen am Unterschenkel auftreten. Bei ausgedehnten Geschwüren, die zirkulär den gesamten Unterschenkel befallen, spricht man von einem Gamaschenulkus.

Eine weitere heute geläufige Einteilung der chronischen venösen Insuffizienz erfolgt nach der CEAP-Klassifikation. Hierzu sei auf die Publikation "Grundlagen der Phlebologie", Kapitel 4.3 "Chronische venöse Insuffizienz" (Herausgeber, R. Rabe, 3. Aufl., S. 111 bis 129) verwiesen.

Dabei können auch die weiteren Bereiche des Strumpfes, wie Fersenabschnitt, Bundabschnitte etc., aus einem elastischen Material bestehen, das dasselbe Material oder ein anderes als im Beinabschnitt sein kann.

Besonders bevorzugt kann bei einem erfindungsgemäßen Strumpf vorgesehen sein, dass der Fersenabschnitt als Pendelferse ausgebildet ist. Grundsätzlich ist jedoch auch eine neben der Pendelferse, die der RAL-Norm RAL-GZ 387 entspricht, eine angeformte Ferse, wobei die Ferse insbesondere keinen Nahtbereich aufweist, sondern durch thermische Verformung des Gewebes erzielt wird, möglich.

Der erste Bundabschnitt kommt im Bereich der Längsgewölbe, insbesondere im Bereich des inneren Längsgewölbes eines Fußes des Trägers oder proximal davon, also hinter dem Gewölbe in Richtung auf die Ferse zu liegen. Der Fersenabschnitt kann dabei vorzugsweise gummielastisch sein. Der Fersenabschnitt kann jedoch auch kompressionslos ausgebildet sein.

Dabei kann nach einer weiteren bevorzugten Ausführungsform vorgesehen sein, dass der Beinabschnitt ausgehend vom Knöchel einen stufenlos degressiven, stufenweise degressiven oder kontinuierlichen Kompressionsverlauf nach proximal besitzt. Durch den degressiven Druckverlauf wird besonders gut dafür Sorge getragen, dass die Venen die bestmögliche Unterstützung für den Transport des venösen Blutes vom Fußbereich zurück zum Herzen erhalten.

Grundsätzlich ist jedoch auch ein konstanter Druckverlauf möglich. Besonders bevorzugt ist ein Kompressionsstrumpf mit einem Druckverlauf gemäß RAL-Norm GZ 387. Insbesondere kann der Strumpf eine Kompressionsklasse gemäß RAL-Norm (RAL-GZ 387) von I bis III aufweisen.

Dabei kann der Norm RAL-GZ 387 entnommen werden, wie der Druck eines Kompressionsstrumpfes auf ein Bein zu bestimmen ist und insbesondere bestimmt die Norm die verschiedenen Messpunkte für einen Kompressions- und Stützstrumpf. Insbesondere kann vorgesehen sein, dass die Messstellen B und B1 im Bereich der Fessel eines menschlichen Beins liegen und der Druck an der Messstelle B 100% sowie z. B. an der Messstelle B1 70 bis 100% des Drucks an der Messstelle B beträgt. Der Fesselbereich wird dabei durch die Messstellen B und B1 eingeschlossen, wobei die Messstelle B einem Punkt am menschlichen Unterschenkel, der unmittelbar über dem Knöchel an dem Punkt mit dem geringsten Umfang des Unterschenkels liegt, entspricht. Die Messstelle B1 entspricht einem Punkt am menschlichen Unterschenkel, der im vorgegebenen Abstand darüber, also proximal beim Übergang der Achillessehne zur Wade, angeordnet ist. Weitere Punkte gemäß Norm sind beispielsweise der Punkt C, nämlich der größte Umfang der Wade sowie der Punkt D, der zwei Fingerbreit unterhalb der Kniekehle liegt. Die Messpunkte F und G liegen im Bereich eines Oberschenkels.

Dabei ist vorgesehen, dass an der Messstelle B1 im Kompressionsstrumpf mit einer Kompressionsklasse II der Druck zwischen 70 und 100% der Messstelle B entspricht. An der Messstelle C soll gemäß einem Kompressionsklasse-II-Strumpf noch 50 bis 80% des Drucks an der Stelle B herrschen und an der Messstelle F oder G, die im Bereich des Oberschenkels liegen, 20 bis 50%.

Strümpfe, die bis zur Messstelle G reichen, werden dabei als sogenannte Schenkelstrümpfe bezeichnet, wohingegen Strümpfe, die bis zum Messpunkt D reichen, entweder als A-D Strümpfe oder als Wadenstrümpfe bezeichnet werden. Der erfindungsgemäße Strumpf kann entweder als Schenkel- oder als Wadenstrumpf ausgebildet werden. Dabei beginnt der Beinabschnitt im Bereich des Sprunggelenks eines Trägers und reicht je nach Ausgestaltung bis zum Knie oder bis in den Oberschenkelbereich hinein.

Dabei ist für die Kompressionsklassen vorgesehen, dass die Klasse I einen Druck von 18 bis 21 mmHg, die Klasse II von 23 bis 32 mmHg, die Klasse III von 34 bis 46 mmHg und die Klasse IV von 49 mmHg und größer aufweist.

Dabei kann bevorzugt vorgesehen sein, dass angrenzend an den Beinabschnitt ein Druckentlastungsbereich nach distal vorgesehen ist. Dieser Druckentlastungsbereich liegt im anwendungsgerechten Zustand des Strumpfes bzw. der Strümpfe im Bereich des Knöchels und ist im Strumpf zwischen dem Beinabschnitt und dem Fersenabschnitt angeordnet. Dieser Druckentlastungsbereich hat den Vorteil, dass angrenzend an den Fesselbereich, insbesondere am Punkt B, der bei einem Strumpf nach RAL-GZ 387 den höchsten Druck aufweist, kein abrupter Übergang von sehr hohem Druck auf niedrigen bis sehr niedrigen Druck oder gar keinen Druck erfolgt und somit keine Abschnürungen innerhalb der Fläche zu verzeichnen sind, die von dem Strumpf bedeckt wird. Der Druckentlastungsbereich dient somit dazu einen ausgleichenden Übergang zwischen zwei Bereichen verschiedenen Drucks bereitzustellen. Ganz besonders bevorzugt ist dabei, wenn der Kompressionsstrumpf eine Pendelferse umfasst, die allseitig angrenzend durch einen Druckentlastungsbereich umschlossen ist. Insbesondere kann der Druckentlastungsbereich in Seitenansicht des Kompressionstrumpfes eine Ypsilon-Form (Y-Form) oder V-Form aufweisen. In dieser Ausgestaltung ist die Pendelferse vollumfänglich von dem Druckentlastungsbereich eingefasst. Es kann jedoch auch vorgesehen sein, dass der Druckentlastungsbereich lediglich am distalen Ende des Beinabschnitts angeordnet ist, an den sich dann die Ferse anschließt. In diesem Fall kann der erste Bundabschnitt unmittelbar an den Fersenabschnitt anschließen, wohingegen für den Fall, dass der Druckentlastungsbereich den Fersenbereich allseitig umschließt, zwischen Fersenbereich und erstem Bundabschnitt ein Bereich des Druckentlastungsbereichs liegt.

Dabei kann vorgesehen sein, dass der erste Bundabschnitt eine Breite von weniger als 3 cm, vorzugsweise 1 bis 2 cm aufweist und somit nur unwesentlich über den Fersenbereich in den Bereich der Längsgewölbe hineinragt bzw. vollständig im Bereich der Längsgewölbe zu liegen kommt.

Dabei kann zusätzlich oder unabhängig vorgesehen sein, dass der Druckentlastungsbereich zwischen der Ferse und dem ersten Bundabschnitt eine Breite von weniger als 2,5 cm, vorzugsweise 1 bis 2 cm aufweist und somit nur unwesentlich über den Fersenbereich in den Bereich der Längsgewölbe hineinragt bzw. vollständig im Bereich der Längsgewölbe zu liegen kommt.

Insbesondere kann der erste Bundabschnitt in der proximalen Hälfte der Längsgewölbe, insbesondere in der proximalen Hälfte des inneren Längsgewölbes zu liegen kommen und besonders bevorzugt im proximalen Drittel der Längsgewölbe, insbesondere im proximalen Drittel des inneren Längsgewölbes zu liegen kommen. In diesem Bereich kann der erste Bundabschnitt besonders gut ein Verrutschen des Strumpfes verhindern.

Des Weiteren kann vorgesehen sein, dass sich an den Beinabschnitt nach proximal ein weiterer Bundabschnitt anschließt, wobei sowohl der erste als auch der weitere Bundabschnitt die Aufgabe haben, einen erfindungsgemäßen Kompressionsstrumpf möglichst sicher, jedoch ohne abzuschnüren und ohne unangenehmes Tragegefühl, am Bein eines Trägers zu fixieren.

Dabei kann der vorgesehen sein, dass der erste und weitere Bundabschnitt auf ihrer dem Träger zugewandten Seite eine ein Verrutschen verhindernde Beschichtung oder ein Haftband, das mit einer solchen Beschichtung versehen ist, aufweisen. Die Bundabschnitte können jedoch auch ohne Haftmittel wie eine Beschichtung ausgebildet sein. Eine derartige Beschichtung kann insbesondere eine Silikonbeschichtung oder auch eine andere Beschichtung sein, wie sie üblicherweise bei Kompressionsstrümpfen oder auch anderen Strümpfen, die halterlos getragen werden, vorgesehen ist, und eine Haftung vorzugsweise dergestalt aufweisen, dass das Haftband auf der Haut des Trägers haftend, jedoch ein Verkleben mit übrigen Teilen der Bekleidung oder dem Strumpf selber vermieden wird, vorgesehen ist. Die Beschichtung kann dabei vorzugsweise in Mustern z. B. in Umfangsrichtung verlaufende Streifen, gitterförmig oder kalottenförmig (punktförmig) aufgebracht sein. Auf diese Weise wird erreicht, dass die elastischen Eigenschaften des Strumpfes auch in diesem Bereich hinreichend erhalten bleiben und darüber hinaus eine Luft- und Wasserdampfdurchlässigkeit in diesem Bereich sichergestellt werden kann.

Nach einer bevorzugten Ausführungsform kann vorgesehen sein, dass ein von distal nach proximal schräger Verlauf an der Oberschenkelinnen- oder -außenseite von posterior nach anterior des weiteren Bundabschnitts vorgesehen ist, wobei dieser Verlauf gekrümmt oder ungekrümmt sein kann. Dies bedeutet, dass bei Betrachtung eines Trägers eines entsprechenden Strumpfes von der Seite des weiteren Bundabschnitts von dessen Rücken zu dessen Bauchseite schräg nach oben verläuft. Der Verlauf ist hierbei unterhalb des Übergangs vom Oberschenkel zum Gesäß, der generell niedriger angeordnet ist in Bezug auf einen am Boden stehenden Träger eines entsprechenden Strumpfes als die Gehfalte zwischen Oberschenkel und Rumpf. Dieser Verlauf kann dabei geschwungen oder ungeschwungen sein, wobei insbesondere der geschwungene Verlauf mit einem Bogen im Bereich der halben Oberschenkelbreite eine besonders anatomische Gestaltung besitzt. Der weitere Bundabschnitt besitzt dann in seiner Seitenansicht eine S-Form. Dabei können die Verläufe des weiteren Bundabschnitts an der Schenkelinnen- und der Schenkelaußenseite desselben Strumpfes voneinander verschieden sein.

Sofern es sich um einen A-D-Strumpf, also um einen Wadenstrumpf handelt, wird dieser in der Regel einen ungeschwungenen und in jedem Bereich des Umfanges gleich hohen weiteren Bundabschnitt aufweisen.

Dabei kann insbesondere vorgesehen sein, dass der Strumpf aus einem elastischen Material, das als Gestrick und vorzugsweise als Rundgestrick ausgebildet ist, besteht.

Insbesondere kann der ganze Strumpf nahtlos ausgeführt sein auch hinsichtlich der Verbindung von Fersen- und Beinabschnitt sowie weitere Abschnitte.

Dabei kann vorgesehen sein, dass es sich bei den verwendeten Materialien um solche handelt, die aus Fäden bestehen, bei denen ein elastischer Kernfaden mit einem weiteren Garn, insbesondere einem Baumwollfaser enthaltenden Garn, als Umwindefaden umwickelt ist. Dabei kann besonders vorteilhaft vorgesehen sein, dass als Kernfaden ein Elastankern, beispielsweise ein Kern aus Lycra® verwendet wird, wobei dieser Kern einfach oder doppelt oder mehrfach umwunden sein kann und wobei das zum Umwinden verwendete Garnmaterial ein Polyamid sein kann oder wahlweise kann ein CoolPlus-Garn der Firma Carl Weiske, Hof, Deutschland, eingesetzt werden kann. Ein entsprechendes Gestrick kann dabei den Vorteil besitzen, dass es nahtlos als Rundgestrick ausgeführt werden kann, wodurch die Trageeigenschaften verbessert werden.

Zusätzlich kann eine 3X-Dry-Ausrüstung der Firma Schoeller, Sevelen, Schweiz, aufgebracht werden, um einen Kühleffekts des verwendeten Garns und damit des verwendeten Kompressionsstrumpfes zu erreichen.

Neben der Verwendung eines Lycra-Fadens können als Strickgarne auch Glattgarne eingesetzt werden, wobei Glattgarne den Vorteil besitzen, durchscheinend zu sein. Auf diese Weise kann eine verbesserte Transparenz oder Transluzenz des Kompressionstrumpfes erreicht werden, wodurch der Strumpf eine kühlere, sommerlichere Erscheinung besitzt und im Sommer lieber getragen wird. Darüber hinaus besitzt ein eher durchscheinender Strumpf auch aufgrund von optischen Gesichtspunkten gerade in den Sommermonaten eine größere Akzeptanz.

Dabei kann das Gestrick auch aus verschiedenen vorzugsweise abwechselnde Strickfäden bestehen, insbesondere können sich Lycra- und Glattgarn-Strickfäden abwechseln.

Als Schussfaden kann ebenfalls ein Lycra®-Material verwendet werden, wobei hier vorgesehen sein kann, dass der Schussfaden nur im Bereich jedes zweiten Strickfadens eingelegt ist. Sofern die vorstehenden Strickgarne verwendet werden, kann der Schussfaden entweder im Bereich des Lycra-Strickgarns Verwendung finden oder im Bereich des Glattgarnstrickgarns. Dabei kann vorgesehen sein, dass die Schussfäden, die auch als Einlegefäden bezeichnet werden, nicht im Fersenbereich vorgesehen sind, wohl aber im Beinabschnitt, dem Druckentlastungsbereich und/oder den Bundabschnitten.

Die Erfindung soll im Folgenden anhand einer Zeichnung näher erläutert werden.

### Dabei zeigen:

- Figur 1: eine schematische Darstellung eines erfindungsgemäßen Kompressionsstrumpfes und
- Figur 2: ein Legebild eines Teils des Gestrickes des Kompressionsstrumpfes.
- Figur 3: eine Darstellung eines Fußes von unten.

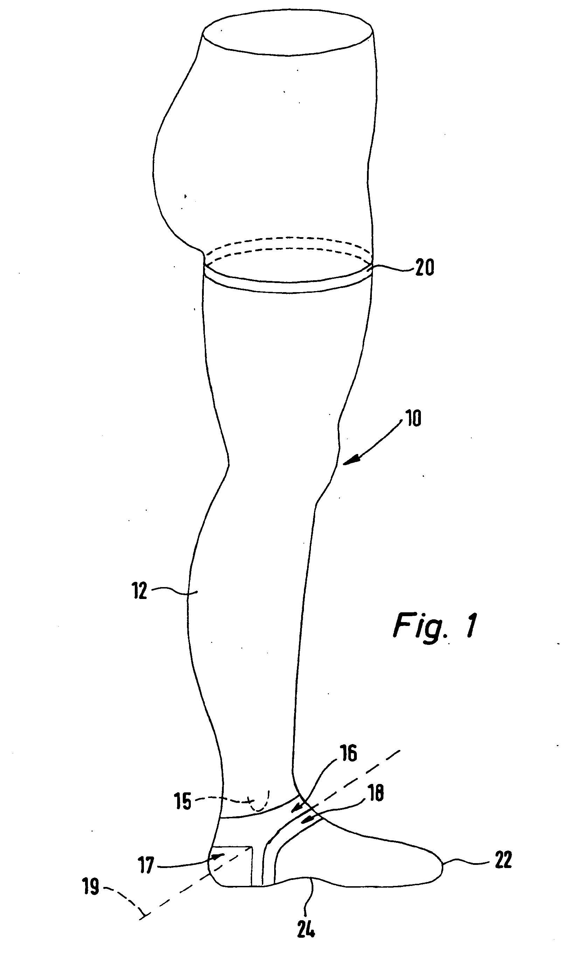

Figur 1 zeigt einen Stütz- oder Kompressionsstrumpf, der gemäß RAL-GZ 387 als Strumpf der Kompressionsklasse II hinsichtlich des Druckverlaufs ausgebildet und in seiner Gesamtheit mit dem Bezugszeichen 10 versehen ist. Dieser weist einen Beinabschnitt 12 auf, der röhrenförmig als Rundgestrick aus einem elastischen Material ausgebildet ist. In Richtung auf den Körper eines Trägers, also nach proximal, schließt der Beinabschnitt 12 mit einem Bundabschnitt 20 ab, der auf seiner Innenseite mit einem beschichteten Haftband, das insbesondere eine Silikonbeschichtung aufweist, versehen ist, so dass ein Rutschen des Kompressionsstrumpfes 10 nach distal verhindert werden kann. Die Beschichtung kann dabei in herkömmlicher Weise ausgestaltet sein, wie es auch sonst bei bereits üblichen halterlosen Strümpfen vorgesehen ist, insbesondere auf dem Gebiet der Kompressionsschenkelstrümpfe. Nach distal an den Beinabschnitt schließt sich ein Druckentlastungsbereich 16 an, der eine Pendelferse 17 vollständig umschließt. Dabei schließt sich sowohl der Druckentlastungsbereich 16 als auch die Pendelferse 17 nahtlos an den Beinabschnitt 12 an. Beinabschnitt 12, Pendelferse 17 und
Druckentlastungsbereich 16 sind insbesondere aus den gleichen Garnen gestrickt. Der Druckentlastungsbereich 16 hat in Abhängigkeit von der Größe der Ferse in der Seitenansicht, wie dargestellt, eine V- oder Y-Form, wobei hier die Y-Form zu sehen ist.

Die sogenannte Y-Linie 19 gemäß der Norm RAL 387 vom September 2000 ist hier mit dem Bezugszeichen 19 versehen. In Richtung auf die Fußspitze 22 schließt sich an den Druckentlastungsbereich 16 ein erster Bundabschnitt 18 an, der entsprechend dem weiteren Bundabschnitt 20 an seiner Innenseite mit einem beschichteten Haftband versehen sein kann, um ein Verrutschen des Kompressionsstrumpfes 10, in diesem Fall insbesondere nach proximal, zu verhindern. Dabei ist der Bundabschnitt 18 so angeordnet, dass er im Bereich der Längsgewölbe eines Fußes oder proximal davon, also in Richtung auf die Ferse, der Längsgewölbe zu liegen kommt. Durch die Anordnung des Bundabschnittes 18 proximal oder im Längsgewölbebereich 24 des Fußes wird erreicht, dass die Fußspitze sowie auch weite Teile des Fußspanns strumpffrei bleiben und es so zum einen nicht zu Abschnürungen im Zehenbereich, wie bei offenen Zehenbereichen vielfach üblich, die als unangenehm durch den Träger empfunden werden, kommen kann und gleichzeitig auch das Verrutschen bzw. Aufrollen des Bundabschnitts 18 weitgehend vermieden wird. Der erste Bundabschnitt 18 besitzt dabei eine Breite von weniger als 3 cm, insbesondere 1-2 cm und liegt in der proximalen Hälfte, bevorzugt im proximalen Drittel der Längsgewölbe 24. Der Druckentlastungsbereich 16 weist zwischen Bundabschnitt 18 und Fersenabschnitt 17 eine Breite von 1-2,5 cm, vorzugsweise 1-2 cm auf und zwischen Fersenabschnitt 17 und Beinabschnitt 12 in etwa die gleichen Abmessungen auf.

Auf diese Weise kann ein Kompressionsstrumpf geschaffen werden, der darüber hinaus auch mit vorne offenen Schuhen im Sommer getragen werden kann und dabei vom Träger zum einen als modisch und zum anderen auch als luftig empfunden wird.

Dabei kann vorgesehen sein, dass der Beinabschnitt 12 einen Druckverlauf gemäß RAL-GZ 387 aufweist, wobei der höchste Druck hier im Bereich eines Messpunktes B1, der im Bereich der schmalsten Stelle der Fessel liegt, auftritt und dann einen degressiven Verlauf in Richtung proximal nimmt. Die entsprechenden Restdruckverhältnisse können bezüglich des Drucks am Punkt B eingestellt werden. Hierdurch soll der Abtransport des venösen Blutes im Bein unterstützt werden.

Der hieran nach distal anschließende Druckentlastungsbereich, der nahtlos an den Beinabschnitt 12 angeformt ist, sorgt dafür, dass es nicht zu einem abrupten und damit einschnürenden Übergang zwischen dem Beinabschnitt 12, insbesondere im Bereich der Fessel bzw. des Knöchels 15 zum Druckentlastungsbereich 16 und damit zum Fuß, kommt. Der Fersenabschnitt 17 ist dabei gummielastisch ausgebildet. Die Bundabschnitte 18 und 20 weisen dabei eine solche Kompression auf, dass ein Abschnüren verhindert ist, aber gleichzeitig ein sicherer Halt gewährleistet werden kann.

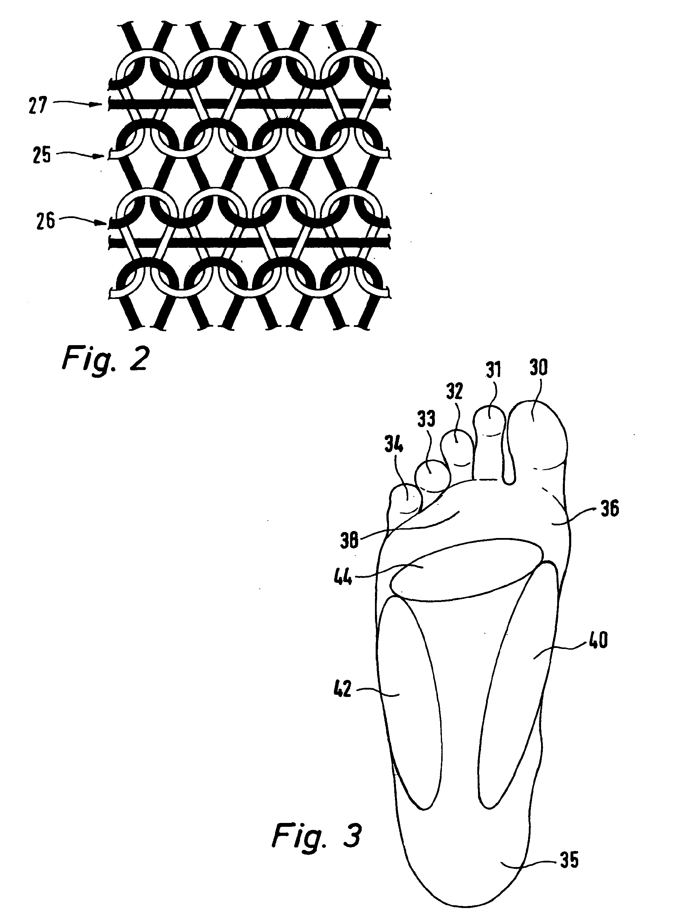

Figur 2 zeigt nun ein Legebild eines Gestricks, wie es in dem erfindungsgemäßen Kompressionsstrumpf 10 eingesetzt ist.

Um eine möglichst gute Transparenz und damit auch eine gefühlte Luftigkeit des Kompressionsstrumpfes 10 zu erreichen, werden insbesondere im Bereich des Beinabschnitts 12, dessen Legebild in Figur 2 gezeigt ist, zwei verschiedene Strickfäden 25 und 26 eingesetzt, die hier durch weiße und schwarze Fäden in Figur 2 symbolisiert sind. Dabei handelt es sich bei dem mit 26 bezeichneten Faden um ein Lycra-Material, das elastisch ausgebildet ist, und insbesondere um Lycra dtex 44 einfach umwunden mit CoolPlus 55 dtex f48/1. Das mit 25 bezeichnete Strickgarn ist dahingegen ein Glattgarn 78 dtex f46. Das Glattgarn ist ein durchscheinendes oder durchsichtiges Garn, wohingegen das Lycra-Material durch das Umwinden mit CoolPlus weniger durchsichtig bis blickdicht erscheint. Der Eintrag des Schussfadens, der hier mit dem Bezugszeichen 27 bezeichnet ist und aus einem Lycra-Material, nämlich Lycra 620 dtex umwunden mit PA 6.6 11 dtex f7/1 besteht, erfolgt lediglich in jeder zweiten Maschenreihe und ist hier im Bereich des Glattgarns 25 vorgesehen. Der Schussfaden 27 ist dabei ebenfalls nicht durchscheinend. Durch die Anordnung des Schussfadens 27 im durchscheinenden Glattgarn 25 ist der optische Eindruck zwischen dem abwechselnden durchscheinenden Strickfaden 25 und dem nicht durchscheinenden Strickfaden 26 weniger auffällig und es kann so ein optisch einheitlicheres Erscheinungsbild entstehen. Alternativ kann der Schussfaden 27 auch im Bereich des Strickfadens 26 vorgesehen sein, so dass dann das Abwechseln von durchscheinenden und nicht durchscheinenden Maschenreihen verstärkt ist.

Darüber hinaus kann vorgesehen sein, dass zusätzlich eine 3X Dry-Ausrüstung der Firma Schoeller, Sevelen, Schweiz für einen weiteren Kühleffekt sorgt.

Figur 3 zeigt nun einen Fuß wobei hier die fünf Zehen 30 bis 34 zu erkennen sind. Die Zehen 30 bis 34 bilden den distalen Abschluss des Fußes wohingegen nach proximal der Fuß durch die Ferse 35 begrenzt ist. Nach proximal anschließend an die Zehen weist der Fuß ein Großzehengrundgelenk 36 sowie Kleinzehengrundgelenke 38 auf, die zusammen als Großzehenballen und Kleinzehenballen den Fußballen bilden. Dabei dient als Aufstandsfläche einer Person auf den Fuß im Wesentlichen die Ferse 35 sowie die Ballen 36 und 38. Zwischen diesen beiden Bereichen, nämlich den Ballen 36 und 38 und der Ferse 35 spannt sich der Fußgewölbebereich. Die Hauptfunktion des Fußes ist dabei das Tragen des Körpergewichts und die Fortbewegung auch über unebene Flächen, d.h. der Fuß muss gleichermaßen Stabilität und Flexibilität bieten. Dabei umfassen die Fußgewölbe das mittlere oder innere Längsgewölbe 40 das oft auch als Hauptgewölbe bezeichnet wird. Es verläuft entlang des Ristes und ist wichtig für die Erhaltung des Gleichgewichts. Es erstreckt sich hierbei zwischen dem Großzehenballen 36 und der Ferse 35. Darüber hinaus verläuft an der Außenkante des Fußes ein weiteres Längsgewölbe, nämlich das seitliche oder äußere Längsgewölbe, manchmal auch als Außengewölbe 42 bezeichnet. Dieses verläuft im Wesentlichen zwischen der Ferse 35 und den Kleinzehenballen, insbesondere des kleinen Zehs 34. Es ist nur ein sehr schwaches Gewölbe, das als Stabilisator für das Gleichgewicht des Fußes fungiert. Im Gegensatz dazu fängt das Hauptgewölbe 40 die Hauptlast der Stöße ab, denen der Fuß ausgesetzt ist. Die Gewölbe 40 und 42 bilden zusammen die Längsgewölbe 24. Weiterhin existiert ein Quergewölbe 44, das im rechten Winkel zu den beiden anderen Gewölben 40, 42 distal hierzu verläuft. Es gibt quer über den ganzen Fuß hinweg Unterstützung und Gleichgewicht. Dieses Gewölbe liegt proximal der Kleinzehenballen 38 sowie des Großzehenballens 36. Proximal hieran schließen sich dann im Wesentlichen die Längsgewölbe 24 an.

Die Fußgewölbe 40 bis 44 werden dabei durch die Muskulatur verspannt und durch Bänder aufrechterhalten, sowie durch die Formen der enthaltenen Knochen sowie der Gelenke die diese miteinander verbinden.

Weitere Vorteile und Merkmale der Erfindung lassen sich dabei den übrigen Anmeldungsunterlagen entnehmen, wobei besonders bevorzugt ist, dass auf diese Weise ein Kompressions- oder Stützstrumpf bereitgestellt werden kann, der eine besonders luftige und damit sommerliche Erscheinung besitzt und insbesondere auch in vorne bzw. zehenoffenen Schuhen getragen werden kann. Darüber hinaus besitzt ein derartiger Strumpf 10 auch den Vorteil bei Patienten mit Fuß- oder Zehenstellungsproblemen sowie Patienten, die beispielsweise aufgrund anderer Erkrankungen, wie Diabetes, keine Kompression im Fuß- oder Zehenbereich vertragen, eingesetzt werden zu können.

Auch besteht der Vorteil, dass ein leichtes Anziehen aufgrund des fehlenden Fußteils möglich ist, da der Strumpf beispielsweise auch gleich "auf rechts" angezogen werden kann und es nicht notwendig ist, ihn auf links gezogen über den Fuß zu ziehen und dann über das Bein nach rechts zu wenden.

Ferner besteht der Vorteil, dass aufgrund der nahtlosen Gestaltung im Fußbereich keine Gefahr von zusätzlichen Druckstellen gegeben ist.

Schließlich ist durch den fehlenden Fußteil auch besonders einfach ein Verbandswechsel im Bundbereich ohne Ablegen des Strumpfes 10 möglich.

## Patentansprüche

1. Kompressions- oder Stützstrumpf (10) zum Anlegen an ein menschliches Bein umfassend einen röhrenförmigen komprimierenden Beinabschnitt (12) aus einem elastischen Material, der das Bein eines Trägers umschließt sowie einen Fersenabschnitt (17), der sich unmittelbar oder mittelbar an den komprimierenden Beinabschnitt (12) distal anschließt, **dadurch gekennzeichnet, dass** ein mittelbar oder unmittelbar an den Fersenabschnitt (17) distal anschließender röhrenförmiger erster Bundabschnitt (18) zur Fixierung der Lage des Kompressions- oder Stützstrumpfes (10) im Bereich eines Fußes des Trägers vorgesehen ist, wobei der erste Bundabschnitt (18) im Bereich der Längsgewölbe (24) oder proximal der Längsgewölbe (24) eines Fußes des Trägers zu liegen kommt.

2. Kompressions- oder Stützstrumpf nach Anspruch 1, **dadurch gekennzeichnet, dass** der Fersenabschnitt (17) als Pendelferse ausgebildet ist.

3. Kompressions- oder Stützstrumpf nach Anspruch 1 oder 2, **dadurch gekennzeichnet, dass** der Beinabschnitt (12) ausgehende vom Knöchel (15) einen stufenlos degressiven, stufenweise degressiven oder kontinuierlichen Kompressionsverlauf nach proximal besitzt und insbesondere der Kompressions- oder Stützstrumpf (10) ein Kompressionsstrumpf mit einem Druckverlauf gemäß RAL-GZ 387 ist.

4. Kompressions- oder Stützstrumpf nach einem der vorangehenden Ansprüche, **dadurch gekennzeichnet, dass** der Fersenabschnitt (17) allseitig durch einen Druckentlastungsbereich (16) umschlossen ist.

5. Kompressions- oder Stützstrumpf nach einem der vorangehenden Ansprüche, **dadurch gekennzeichnet, dass** sich an den Beinabschnitt (12) nach proximal ein weiterer Bundabschnitt (20) anschließt.

6. Kompressions- oder Stützstrumpf nach einem der vorangehenden Ansprüche, **dadurch gekennzeichnet, dass** der erste und/oder weitere Bundabschnitt (18, 20) auf ihrer dem Träger zugewandten Seite eine, ein Verrutschen verhindernde Beschichtung oder ein Haftband, das mit einer solchen Beschichtung versehen ist, aufweisen.

7. Kompressions- oder Stützstrumpf nach einem der vorangehenden Ansprüche, **dadurch gekennzeichnet, dass** der erste Bundabschnitt (18) eine Breite von weniger als 3 cm, vorzugsweise von 1 bis 2 cm aufweist.

8. Kompressions- und Stützstrumpf nach einem der vorangehenden Ansprüche, **dadurch gekennzeichnet, dass** der erste Bundabschnitt (18) in der proximalen Hälfte, vorzugsweise dem proximalen Drittel der Längsgewölbe (24) zu liegen kommt.

9. Kompressions- oder Stützstrumpf nach einem der vorangehenden Ansprüche, **dadurch gekennzeichnet, dass** das elastische Material ein Gestrick, vorzugsweise ein Rundgestrick ist.

10. Kompressions- oder Stützstrumpf nach einem der vorangehenden Ansprüche, **dadurch gekennzeichnet, dass** der Strumpf (10) nahtlos ist.

11. Kompressions- oder Stützstrumpf nach einem der vorangehenden Ansprüche, **dadurch gekennzeichnet, dass** der Strumpf (10) ein Kompressionsstrumpf (10) gemäß einer der Kompressionsklassen I-III gemäß RAL-GZ 387 ist.

12. Kompressions- oder Stützstrumpf nach einem der vorangehenden Ansprüche, **dadurch gekennzeichnet, dass** der Strumpf (10) ein Schenkel- oder Wadenstrumpf ist.

13. Kompressions- oder Stützstrumpf nach einem der vorangehenden Ansprüche, **dadurch gekennzeichnet, dass** Strumpf (10) durchscheinend ist.

14. Kompressions- oder Stützstrumpf nach einem der vorangehenden Ansprüche, **dadurch gekennzeichnet, dass** als Strickgarne (25, 26) abwechselnd verschiedene Garntypen, insbesondere Glattgarne und Lycra eingesetzt werden.

15. Kompressions- oder Stützstrumpf nach einem der vorangegangenen Ansprüche, **dadurch gekennzeichnet, dass** ein Schussfaden (27) nur im Bereich jedes zweiten Strickfadens (25, 26) eingelegt ist.
